Europäisches Patentamt

European Patent Office

Office européen des brevets

(19)

(11) Veröffentlichungsnummer: **0 021 470**
**B1**

(12) ## EUROPÄISCHE PATENTSCHRIFT

(45) Veröffentlichungstag der Patentschrift:
**09.11.83**

(21) Anmeldenummer: **80200463.0**

(22) Anmeldetag: **19.05.80**

(51) Int. Cl.³: **B 01 F 13/02** // C12M1/06

(54) **Hohlrührer zum Eintragen von Gas in eine Flüssigkeit.**

(30) Priorität: **01.06.79 CH 5124/79**

(43) Veröffentlichungstag der Anmeldung:
**07.01.81 Patentblatt 81/1**

(45) Bekanntmachung des Hinweises auf die Patenterteilung:
**09.11.83 Patentblatt 83/45**

(84) Benannte Vertragsstaaten:
**AT CH DE FR GB IT LI SE**

(56) Entgegenhaltungen:
AT - B - 189 148
AT - B - 193 331
DE - A - 2 207 144
DE - C - 863 656
GB - A - 2 001 252

(73) Patentinhaber: **Chemap AG, Alte Landstrasse 415, CH-8708 Männedorf (CH)**

(72) Erfinder: **Sotirianos, Konstantin, Ebnetstrasse 17a, CH-8712 Stäfa (CH)**

(74) Vertreter: **Herrmann, Peter, Chemap AG Alte Landstrasse 415, CH-8708 Männedorf (CH)**

## Hohlrührer zum Eintragen von Gas in eine Flüssigkeit

Gegenstand der Erfindung ist ein Hohlrührer zum Eintragen von Gas in eine Flüssigkeit, insbesondere die Eintragung eines sauerstoffhaltigen Gases zur aeroben Züchtung von Mikroorganismen.

Hohlrührer sind hohl ausgebildete Rührkörper, deren Innenraum über eine Hohlwelle einerseits mit einem Gasraum und andererseits mit einer Flüssigkeit in Verbindung stehen. Während der Rotation des Rührkörpers wird Gas nach dem Strahlsaugerprinzip angesaugt.

Nach dem deutschen Gebrauchsmuster Nr. 7 228 282 ist ein Hohlrührer der Art bekannt. Darin sind die Rohre der Rührarme, der Drehrichtung vorauseilend, abgebogen. Diese Konstruktion ist strömungsungünstig, wodurch aber offenbar ein starker Sog bewirkt wird. Nachteil solcher Konstruktionen ist bei gutem Gasdurchsatz ein verhältnismäßig hoher Energieaufwand.

Die DE-C-863 656 beschreibt eine Begasungsvorrichtung mit Gasaustrittsöffnungen in Form von Rohrstutzen, die nicht mit einer Hohlwelle verbunden sind. In der DE-A-2 207 144 sind schlitzartige Gasaustrittsöffnungen mit relativ großer Fläche und in der AT-A-193 331 sind U-förmige, ebene Frontflächen aufweisende bzw. stromlinienförmige Profile als Begasungsmittel angegeben. In M. Zlokarnik, Chem. Ing. Techn. 38, 357 (1966), wird ein Rohrrührer beschrieben, dessen gerade Rohre an den Enden schräg abgeschnitten sind, um einen großen Sog erzeugende Kante zu bilden.

Alle diese bekannten Hohlrührer haben den Nachteil, daß Einbauten in den Rührarmen oder aufwendige Gestaltungen der Rührarme als notwendig erachtet werden, um den Gasdruck bzw. den Gasstrahl gerichtet in die Flüssigkeit zu leiten, um so den gewünschten Gaseintrag zu erreichen. Hohlrührer der bekannten Art sind auch zur Züchtung von Mikroorganismen unter sterilen Bedingungen wenig geeignet, da eine Reinigung und Sterilisierung der komplizierten Rührer mit vielen Ecken, Kanten und Schweißstellen nur unter großem Aufwand einer Sterilisierung zugänglich sind.

Aufgabe der Erfindung ist die Schaffung eines Hohlrührers, der mit möglichst niedrigem Energieaufwand eine möglichst gute Begasung einer Flüssigkeit, insbesondere einer Fermentationsbrühe, erreicht.

Diese Aufgabe wird erfindungsgemäß mit dem Hohlrührer nach Patentanspruch 1 gelöst. Der Hohlrührer ist dadurch gekennzeichnet, daß die Rührarme des Hohlrührers aus gleichmäßig gekrümmten 90°-Rohrbogen mit an den Enden kreisförmigen Austrittsöffnungen gleichen Durchmessers wie die Rohrbogen gebildet sind.

Die Rohrbogen können zwischen einer feststehenden oberen Ringscheibe und einer unteren Ringscheibe befestigt sein, und sie können unter einem Winkel von 30° zur Radialen der Drehachse des Hohlrührers versetzt sein. Es können zwei oder mehrere gegenständige Rohrbogen die Rührarme bilden. Am äußeren Rand der unteren Ringscheibe können Prallbleche befestigt sein.

In überraschender Weise hat sich gezeigt, daß einfache, gleichmäßig gekrümmte Rohrbogen mit kreisrunden Austrittsöffnungen, ohne Einbauten, bei relativ geringem Energieaufwand, eine hohe Gaseintragsleistung erbringen. Der Einsatz von Normrohrbogen stellt eine preiswerte Lösung dar.

Die Erfindung soll anhand von Zeichnungen erläutert werden. Es zeigt

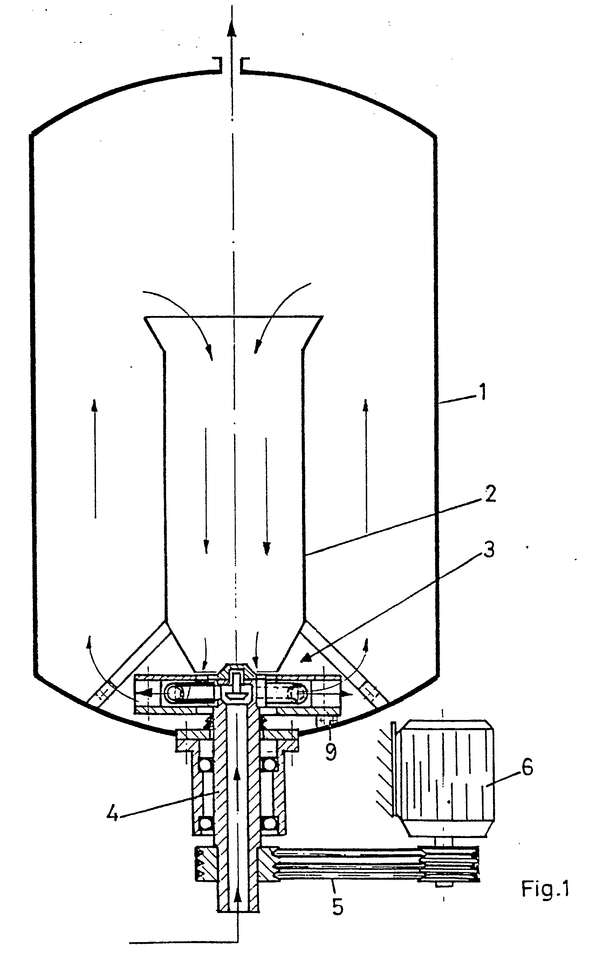

Fig. 1 die erfindungsgemäße Vorrichtung in einem Begasungsreaktor,

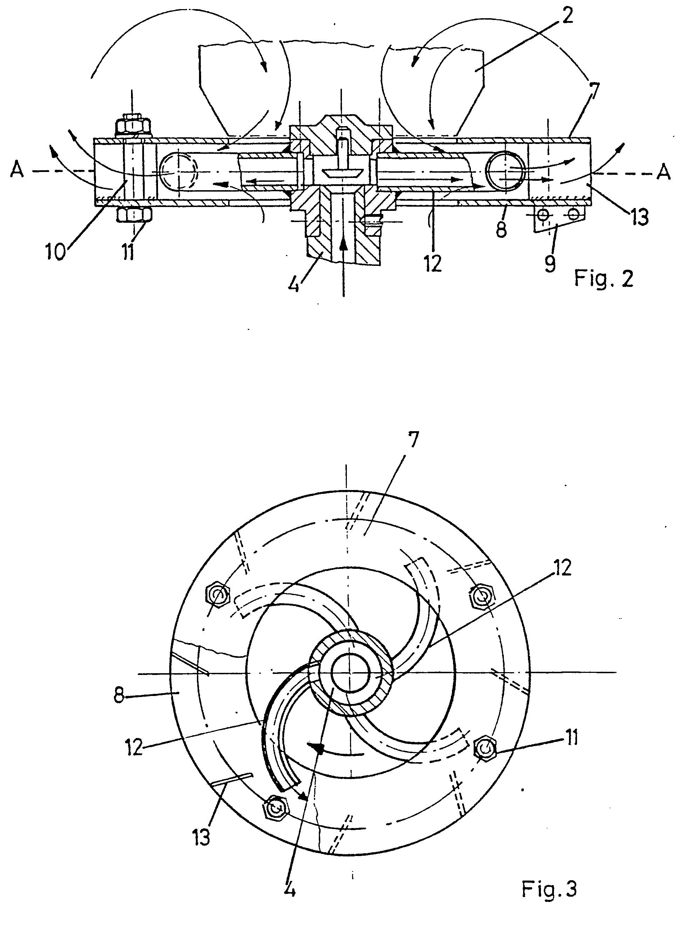

Fig. 2 die Rührvorrichtung im Längsschnitt,

Fig. 3 die Rührvorrichtung der Fig. 2 im Querschnitt A-A.

In einem Reaktorbehälter 1 ist ein zentrales Leitrohr 2 eingebaut. Am unteren Ende des Leitrohres 2 ist das Belüftungsaggregat 3 mit einer Hohlwelle 4 verbunden. Die Hohlwelle 4 ist über einen Antrieb 5 mit einem Motor 6 verbunden. Das Belüftungsaggregat 3 wird aus einem Stator gebildet, der aus einer oberen Ringscheibe 7 und einer unteren Ringscheibe 8 besteht und so eine Öffnung zwischen den Ringscheiben und der Hohlwelle 4 bildet. Der Stator ist über eine Befestigung 9 am Kesselboden des Behälters 1 angebracht. Auf der Gegenseite der Befestigung 9 werden die Ringscheiben 7, 8 des Stators durch Bolzen 10 mittels Muttern 11 zusammengehalten. Die Rührarme bestehen aus zwei oder mehreren Rohrbogen 12, die in gleichen Abständen voneinander auf der Hohlwelle befestigt sind. Die Öffnungen der Rohrbogen befinden sich etwa in der Mitte des Stators und werden somit durch die Ringscheiben 7 und 8 abgedeckt. Die Rohrbogen 12 bestehen aus 90°-Bogen, so daß der Bogen einen Viertelkreis darstellt. Die Rohrbogen 12 sind nicht senkrecht auf der Hohlwelle 4 befestigt, sondern unter einem Winkel zur Senkrechten von vorzugsweise 30°. Zwischen den Ringscheiben sind auf deren äußerem Rand Prallbleche angeordnet, die nur auf der Ringscheibe 8 befestigt sind.

Im Betrieb wird das Belüftungsaggregat in Form eines Hohlrührers 3 über die Hohlwelle 4 und Getriebe 5 vom Motor 6 angetrieben. Dabei wird Flüssigkeit aus dem Behälter 1 durch Öffnungen der oberen Ringscheibe 7 und unteren Ringscheibe 8 angesaugt und über die Rohrbogen 12 nach außen gemäß der Pfeile geschleudert. Dabei entsteht ein Unterdruck in der Hohlwelle 4, wobei Luft angesaugt und der Flüssigkeit beigemischt wird. Die Öffnungen an den Enden der Rohrbogen 12 weisen dabei in die der Drehung entgegengesetzte Richtung. Es hat sich gezeigt, daß eine Umwälzung mit 90°-Rohrbogen unter gleichzeitiger Belüftung besonders wirkungsvoll ist. Dabei verhindern die um ca. 30° angestellten Prallbleche 13 eine Rotation innerhalb der Belüftungsaggregate 3 und begünstigen so eine sehr starke Durchmischung der Flüssigkeit mit dem Gas.

Meßergebnisse:

Rohrbogen-Belüfter ⌀ 370 mm (6 Rohrbögen NW 40)
Motorleistung: 10 kW
Motor: 10 kW, 1460 rpm
Drehzahl des Belüfters: 1000 rpm
Luftansaug (Nm³/h) und Energiebedarf (kW/Nm³) in Abhängigkeit vom hydrostatischen Druck.

| H | Q | P | E |
|---|---|---|---|
| (mWS) | (Nm³/h) | (kW) | (kW/Nm³) |
| 1,00 | 140,00 | 8,00 | 0,057 |
| 2,00 | 114,00 | 12,30 | 0,108 |
| 3,00 | 106,50 | 15,00 | 0,141 |
| 4,00 | 100,20 | 17,50 | 0,175 |

Die Vorrichtung ist insbesondere zur aeroben Züchtung von Mikroorganismen geeignet. Sie kann aber auch bei jeder anderen Gas-Flüssigkeit-Reaktion eingesetzt werden, die eine intensive Begasung erforderlich macht. Auch auf dem Gebiet der Abwasserreinigung ist dieses Belüftungssystem geeignet.

Legende: H = Höhe der Flüssigkeitssäule
Q = Luftmenge
P = aufgenommene Leistung
E = Energiebedarf pro m³

## Patentansprüche

1. Hohlrührer zur Begasung einer Flüssigkeit, insbesondere zum Eintragen eines sauerstoffhaltigen Gases, zur aeroben Züchtung von Mikroorganismen, mit einer hohlen Antriebswelle und als Gaszuleitung dienenden hohlen Rührarmen, die zur Drehrichtung entgegengesetzt gebogen sind und in einer Ebene senkrecht zur Drehachse angebracht sind, dadurch gekennzeichnet, daß die Rührarme (12) des Hohlrührers (3) aus gleichmäßig gekrümmten 90°-Rohrbogen mit an den Enden kreisförmigen Austrittsöffnungen gleichen Durchmessers wie die Rohrbogen gebildet sind.
2. Hohlrührer nach Anspruch 1, dadurch gekennzeichnet, daß die Rohrbogen (12) zwischen einer feststehenden oberen Ringscheibe (7) und einer feststehenden unteren Ringscheibe (8) befestigt sind.
3. Hohlrührer nach Anspruch 2, dadurch gekennzeichnet, daß die Rohrbogen (12) unter einem Winkel von 30° zur Radialen der Drehachse des Hohlrührers angebracht sind.
4. Hohlrührer nach den Ansprüchen 1 bis 3, dadurch gekennzeichnet, daß die Rührarme (12) aus zwei oder mehreren gegenständigen Rohrbogen bestehen.
5. Hohlrührer nach den Ansprüchen 1 bis 4, dadurch gekennzeichnet, daß Prallbleche (13) auf dem äußeren Rand der unteren Ringscheibe (8) befestigt sind.

## Claims

1. A hollow stirrer for gasification of a liquid medium particularly for introducing an oxygen-containing gaseous medium for the aerobic cultivation of microorganisms, including a hollow shaft, and hollow tube members serving simultaneously as gas supply pipes, which are inclined in a direction opposite to that of the rotation and which are fixed at a right angle to the axis of rotation, characterized in that the tube members (12) of the hollow stirrer (3) are of uniformly curved tubes of a curvature defined by an arc of 90° sector, the peripheral annular outlet apertures are of the same diameter as the curved tubes themselves.
2. The hollow stirrer of claim 1, characterized in that the tube members (12) are rigidly connected between an upper annular ring (7) and a lower annular ring.(8).
3. The hollow stirrer of claim 2, characterized in that the tube members (12) are inclined at 30° to the outer surface of the shaft (4).
4. The hollow stirrer of the claims 1 to 3, characterized in that the tube members (12) are of two or a plurality of opposite tube members.
5. The hollow stirrer of the claims 1 to 4, characterized in that flat baffles (13) are positioned on the outer rim of the lower annular ring (8).

## Revendications

1. Agitateur creux, pour l'introduction de gaz dans un liquide, en particulier pour l'introduction d'un gaz oxygéné en vue d'une culture aérobie de microorganismes; au moyen d'un arbre de transmission creux et des rames d'agitateur creux utilisées comme conduits à gaz et qui sont recourbées dans le sens opposé du sens de rotation et fixées perpendiculiairement à l'axe de l'arbre de transmission; caractérisé en ce que les tubes recourbés (12) de l'agitateur creux (3) sont constitués tubes croux uniformément recourbés suivant un arc de 90° (12) et dont les extrémités comportent des sorties circulaires de même diamètre que le diamètre intérieur des tubes recourbés (12) présentant de même rayon de courbure.
2. Agitateur creux selon la revendication 1, caractérisé en ce que les tubes recourbés (12) sont montés entre un disque annulaire supérieur fixe (7) et un disque annulaire inférieur fixe (8).

3. Agitateur creux selon la revendication 2, caractérisé en ce que les tubes recourbés (12) sont montés décalés d'un angle de 30° par rapport à la ligne radiale vers l'axe de l'agitateur creux.

4. Agitateur creux selon les revendications 1 à 3, caractérisé en ce que les tubes recourbés (12) se compose de deux ou plusieur tubes recourbés opposés.

5. Agitateur creux selon les revendications 1 à 4, caractérisé en ce que des tôles de chicane (13) sont fixées sur le bord extérieur du disque annulaire inférieur (8).

Fig.1

Fig. 2

Fig. 3